# EUROPEAN PATENT APPLICATION

(11) **EP 4 194 183 A1**
(43) Date of publication of application: **14.06.2023**
(21) Application number: 21212697.3
(22) Date of filing: 07.12.2021
(51) Int. Cl.: B29C 65/08, B29C 69/00, A61F 13/15, B29L 31/48

(54) **UNIT, MACHINE AND METHOD FOR MANUFACTURING SANITARY PADS**

(71) Applicant: Fameccanica.Data S.p.A., 66020 San Giovanni Teatino (CH) (IT)
(72) Inventor: CETRA, Paolo, I-66020 San Giovanni Teatino (Chieti) (IT)
(74) Representative: Marchitelli, Mauro

(57) **Abstract**

A forming unit for manufacturing a component of sanitary pads (S) and the like comprising a number of superimposed layers, including means for feeding a continuous web forming a transfer layer (P) and a continuous web forming a topsheet (T) along a production path to a coupling station (10) including a cutting device (18) for intermittently cutting the transfer layer (P) to provide spaced-apart transfer layer patches (Pp), a first roller (14) and an associated first ultrasound welding device (20) to partially spot weld said transfer layer patches (Pp) onto the topsheet (T) around the first roller (14), and a second roller (16) and an associated second ultrasound welding device (22) to complete welding of the transfer layer patches (Pp) to the topsheet (T) around the second roller (16). The first and second rollers (14, 16) are carried by a common frame structure (12) and are arranged close to each other.

## Description

### Field of the invention

The present invention is generally related to manufacturing sanitary pads and the like comprising a number of superimposed layers.

More specifically the invention is directed to producing a component for the production of such sanitary pads including a continuous web designed to form a topsheet, and patches of a transfer layer secured at set intervals on the topsheet. Such a component is produced at a forming unit and then delivered to subsequent work stations for completing, by adding and fixing additional layers, the manufacturing of the sanitary pads.

### State of the prior art

The present technique for producing such a component for sanitary pads include the steps of feeding a continuous web forming the transfer layer and a continuous web forming the topsheet along a production path to the forming unit, which includes a rotary cutting device and a first roller and associated first welding device. The transfer layer is intermittently cut by the cutting device so as to provide spaced-apart transfer layer patches which are laid onto the topsheet around the first roller and partially spot welded thereto, only at the front ends of the patches with respect to the direction of displacement of topsheet along the production path, the by the first welding device. The topsheet with the partially welded transfer layer patches are then delivered to a second roller and associated second welding device performing a second spot welding so as to complete welding of the transfer layer patches to the topsheet.

The component thus produced is then delivered to subsequent workstations for adding and fixing the further layers required for the manufacturing the sanitary pads.

According to the prior art the first roller together with the rotary cutter and the associated first welding device on one hand, and the second roller along with the associated second welding device on the other hand consist of respective separate assemblies, spaced-apart from each other and carried by separate supporting frames. As a consequence the path of the topsheet between the two spaced-apart assemblies is relatively long, whereby stability of the partially welded transfer layer patches is jeopardized, involving the risk that the patches may become folded onto themselves or lifted up prior to reaching the second welding device, thus causing low quality finished sanitary pads which need to be discarded.

Additionally, when the machine has to be converted to the production of pads having different sizes or features, both assemblies must be removed and replaced thus requiring relative complex operations and relatively long production stops.

### Object and summary of the invention

The object of the present invention is to overcome one or more of the problems of the prior art.

According to the present invention this object is achieved by a unit for manufacturing sanitary pads as set forth in claim 1 and in the subclaims depending thereupon.

According to another aspect the present invention is directed to a machine for manufacturing sanitary pads as recited in claim 7 and in the subclaims depending thereupon.

According to a further aspect, the present invention relates to a method for manufacturing a component of sanitary pads for ladies such as defined in claim 13 and in the subclaims depending thereupon.

The claims form an integral part of the technical disclosure provided here in relation to the invention.

### Brief description of the drawings

The present invention will now be described in detail with reference to the attached drawings, given purely by way of non-limiting example, wherein:
- Figure 1 is a schematic plan view of a sanitary pads for ladies comprising a component including a topsheet segment and a patch of a transfer layer joined together according to the invention,
- Figure 2 shows a variant of Figure 1,
- Figure 3 is a diagrammatic side view of a forming unit of a machine for manufacturing the component of the sanitary pad of Figure 1, and
- Figure 4 is a variant of Figure 3.

### Detailed description

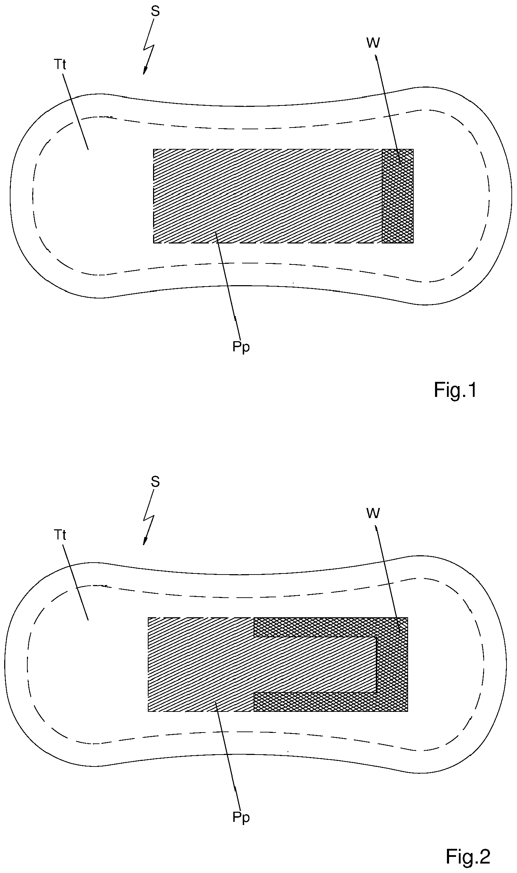

Referring initially to Figure 1 a sanitary pad or diaper S for ladies or the like, such as for instance a bandaid, consists of a layered body comprising in a way known per se a patch Pp of a transfer layer P (Figure 2) welded to a longitudinal segment Tt of a topsheet T (Figure 2). Both the transfer layer P and the topsheet T consist of a continuous web and also in a way known per se the pad S is including a number of additional superimposed absorbent layers as well as an impermeable back sheet, all joined together.

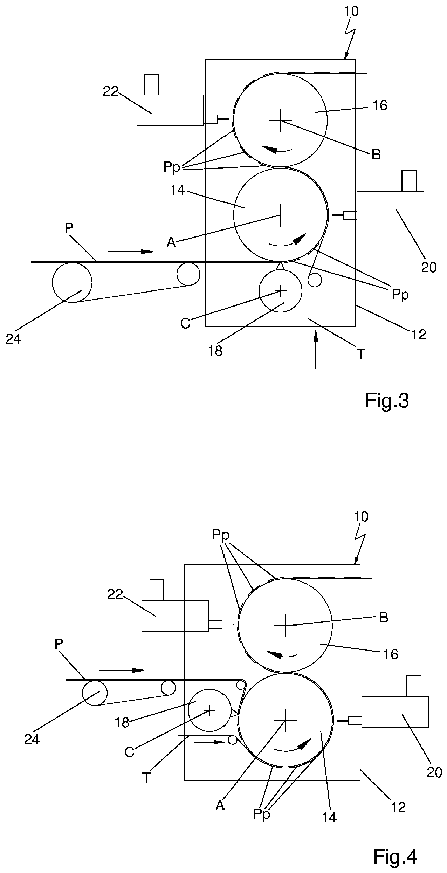

Referring now to Figure 3 reference numeral 10 generally designates a forming unit of a machine according to the invention for the production of a continuous web formed by the topsheet T to which patches Pp of the transfer layer P are consecutively secured at set even intervals, as disclosed herebelow.

The forming unit 10 includes a single support frame 12 bearing both horizontal axes A and B of a first or lower roller 14 and of a second or upper roller 16 which are vertically superimposed and immediately close, i.e. nearly tangent, to each other.

A rotary cutter 18 is operatively associated to the first roller 14, beneath it and with its axis C arranged in a vertical plane containing the axes A and B.

A first ultrasound welding device 20 is also operatively associated to the first roller 14, at one side thereof, and a second ultrasound welding device 22 is operatively associated to the second roller 16, at the opposite side. Both welding devices 20 and 22 conveniently consist of respective sonotrodes and are arranged substantially horizontally, i.e. substantially radially with respect to rollers 14 and 16, or even with a slight inclination.

The rollers 14, 16 are provided, also in a way known per se over angular portions of their surfaces with relief patterns for the spot welding operated by the sonotrodes 20 and 22.

In operation the transfer layer P is supplied, for instance under vacuum on a lateral endless belt 24, between the first roller 14 and the cutter 18, while the topsheet T is fed from below to the roller 14 so as to enclose thereagainst the patches Pp intermittently cut by the cutter 18 from the transfer layer P. The patches Pp are then partially spot welded by the first sonotrode 20: namely, only the front ends thereof with respect to the machine direction, as indicated as W in Figure 1, or even the front ends and adjacent portions of the lateral edges thereof as also indicated as W in Figure 2, are welded to the topsheet T. Since the patches Pp are facing towards the relief pattern areas of roller 14, no substantially visible convex embossing on the topsheet T is generated upon welding.

The topsheet T along with the partially welded patches Pp proceeds to the second roller 16 passing through the almost tangent areas thereof, and reaches the second sonotrode 22 which welds the entire perimeter of every patch Pp on the topsheet T. When spot welding is performed by the first welding device 20 the transfer layer patches Pp are set at the inside of the topsheet T, while when spot welding is performed by the second welding device 22 the transfer layer patches Pp are set at the outside of said topsheet. Since the patches Pp are thus exposed outwardly with respect to the roller 16, the visible embossing generated on the topsheet T by the relief area of roller 16 due to the welding spots is concave. This provides an appreciable advantage in connection with the aesthetic design of the sanitary pads as finally produced.

Further advantages of the invention over the prior art are as follows:
- since the rollers 14 and 16 are set on the common support frame 12 and are close and tangent to each other, stability of the process is greatly enhanced by virtue of that any risks is prevented that the patches Pp partially welded to the topsheet T by the first welding device 20 may become folded or lifted up along the path between the two welding devices 20, 22;
- the single support frame 12 enables relatively easy and rapid removal and replacing of entire the unit 10 for the production of pads having different sizes or features;
- the substantially horizontal arrangement of the two sonotrodes 20, 22 at opposite sides with respect to the axes A, B of rollers 14, 16, the arrangement of the axes A, B and C on a common vertical plane, and the rollers 14 and 16 being tangent to each other allow safe unloading on the support frame 12 of any vibrations induced by the sonotrodes 20, 22.

In the variant depicted in Figure 4, wherein similar or identical parts to those already disclosed with reference to the embodiment of Figure 3 are indicated by the same numerals, the rotary cutter 18 is located aside i.e. laterally to the first roller 14, and both the transfer layer P and the topsheet T are supplied to the first roller 14 laterally, respectively above and below the cutter 18.

Of course, without prejudice to the principle of the invention, the details of construction and the embodiments can be widely varied with respect to those described and illustrated, without thereby departing from the scope of the invention as defined by the claims that follow. Namely, the sonotrodes 20, 22 could be replaced by different devices such as heat weld or laser or mechanical welding devices.

## Claims

1. A forming unit for manufacturing a component of sanitary pads (S) and the like comprising a number of superimposed layers, including means for feeding a continuous web forming a transfer layer (P) and a continuous web forming a topsheet (T) along a production path to a coupling station (10) including a cutting device (18) for intermittently cutting the transfer layer (P) to provide spaced-apart transfer layer patches (Pp), a first roller (14) and an associated first ultrasound welding device (20) to partially spot weld said transfer layer patches (Pp) onto said topsheet (T) around said first roller (14), and a second roller (16) and an associated second ultrasound welding device (22) to complete welding of said transfer layer patches (Pp) to said topsheet (T) around said second roller (16), **characterized in that** said first and second rollers (14, 16) are carried by a common frame structure (12) and are arranged close to each other.

2. Unit according to claim 1, **characterized in that** said first and second rollers (14, 16) are vertically superimposed to each other and said first and second ultrasound welding devices (20, 22) and arranged substantially orizontally and radially with respect to said first and second rollers (14, 16) at opposite sides thereof.

3. Unit according to claim 1 or 2, **characterized in that** said cutting device is a rotary cutter (18) carried by said frame structure (12) and co-operating with said first roller (14).

4. Unit according to claim 3, **characterized in that** rotary cutter (18) is arranged beneath said first roller (14) .

5. Unit according to claim 4, **characterized in that** said first and second rollers (14, 16) and said rotary cutter (18) have respective axes (A, B, C) contained in a common vertical plane.

6. Unit according to claim 3, **characterized in that** said rotary cutter (18) is arranged aside said first roller (14).

7. A machine for manufacturing sanitary pads (S) comprising a number of superimposed layers, including means for feeding a continuous web forming a transfer layer (P) and a continuous web forming a topsheet (P) along a production path to a forming unit (10) including a cutting device (18) for intermittently cutting the transfer layer (P) so as to provide spaced-apart transfer layer patches (Pp), a first roller (14) and an associated first welding device (20) to partially spot weld said transfer layer patches (Pp) onto said topsheet (T) moving around said first roller (14), and a second roller (16) and an associated second welding device (22) to complete welding of said transfer layer patches (Pp) to said topsheet (T) moving around said second roller (16), **characterized in that** said first and second rollers (14, 16) are carried by a common frame structure (12) and are arranged close to each other.

8. Machine according to claim 7, **characterized in that** said first and second rollers (14, 16) are vertically superimposed to each other and said first and second welding devices (20, 22) and arranged horizontally and radially with respect to said first and second rollers (14, 16) at opposite sides thereof.

9. Machine according to claim 7 or 8, **characterized in that** said cutting device is a rotary cutter (18) carried by said frame structure and co-operating with said first roller (14).

10. Machine according to claim 9, **characterized in that** said rotary cutter (18) is arranged beneath said first roller (14).

11. Machine according to claim 10, **characterized in that** said first and second rollers (14, 16) and said rotary cutter (18) have respective axes (A, B, C) contained in a common vertical plane.

12. Machine according to claim 9, **characterized in that** said rotary cutter (18) is arranged laterally to said first roller (14).

13. A method for manufacturing sanitary pads (S) comprising a number of superimposed layers, including the steps of feeding a continuous web forming a transfer layer (P) and a continuous web forming a topsheet (T) along a production path in a machine according to claim 7 including a cutting device (18) and a first roller (14) and associated first welding device (20), intermittently cutting the transfer layer (P) by means of said cutting device (18) so as to provide spaced-apart transfer layer patches (Pp), laying the transfer layer patches (Pp) onto said topsheet (T) moving around said first roller (14), performing a first spot welding by means of said first welding device (20) in order to partially weld said transfer layer patches (Pp) to the topsheet (T), feeding the topsheet (T) to a second roller (16) and associated second welding device (22), and performing a second spot welding by means of said second welding device (22) so as to complete welding of said transfer layer patches (Pp) to said topsheet (T), **characterized in that** said second welding is carried out immediately following said first welding.

14. Method according to claim 13, **characterized in that** the topsheet (T) together with said transfer layer patches (Pp) is passed between said first and second rollers (14, 16) which are immediately close to each other, said first welding being carried out at one side of the first roller (14) and said second welding being carried out at the opposite side of said second roller (16) .

15. Method according to claim 13 or 14, **characterized in that** said first welding is carried out while said transfer layer patches (Pp) are exposed towards said first roller (14) and said second welding is carried out while said transfer layer patches (Pp) are exposed to the outside of said second roller (16).
